# EUROPÄISCHE PATENTSCHRIFT

(11) **EP 0 938 459 B1**
(45) Veröffentlichungstag und Bekanntmachung des Hinweises auf die Patenterteilung: **31.07.2002**
(21) Anmeldenummer: 97950185.5
(22) Anmeldetag: 13.11.1997
(51) Int. Cl.: C07B 37/04, C07C 67/343, C07C 69/618

(54) **VERFAHREN ZUR HERSTELLUNG VON AROMATISCHEN OLEFINEN MITTELS KATALYSE DURCH PALLADAPHOSPHACYCLOBUTANE**
PROCESS FOR PREPARING AROMATIC OLEFINS BY USING PALLADAPHOSPHACYCLOBUTANES AS CATALYSTS
PROCEDE DE PREPARATION D'OLEFINES AROMATIQUES PAR CATALYSE AU MOYEN DE PALLADAPHOSPHACYCLOBUTANES

(30) Priorität: 18.11.1996 DE 19647582
(43) Veröffentlichungstag der Anmeldung: 01.09.1999
(73) Patentinhaber: Degussa AG, 40474 Düsseldorf (DE)
(72) Erfinder: GEISSLER, Holger, D-55116 Mainz (DE); GROSS, Peter, D-65451 Kelsterbach (DE); GUCKES, Bianca, D-65529 Waldems (DE); KLIMPEL, Michael, D-83435 Bad Reichenhall (DE)
(86) Internationale Anmeldenummer: EP9706328
(87) Internationale Veröffentlichungsnummer: WO9822412

(56) Entgegenhaltungen:
- EP-A- 0 725 049
- DE-C- 4 421 730
- W. A. HERRMANN: "Palladacycles as structurally defined catalysts for the Heck olefination of chloro- and bromoarenes" ANGEWANDTE CHEMIE INTERNATIONAL EDITION., Bd. 34, Nr. 17, 15.September 1995, WEINHEIM DE, Seiten 1844-1848, XP002056984

## Beschreibung

Die vorliegende Erfindung betrifft ein neues Verfahren zur Herstellung von aromatischen Olefinen mit Palladaphosphacyclobutanen als neuartigen Katalysatoren.

Aromatische Olefine, insbesondere Zimtsäurederivate, Styrole und Stilbene haben technische Bedeutung als Feinchemikalien, Ausgangsprodukte für Polymere, UV-Absorber und Synthesevorprodukte.

Eine häufig angewandte Methode zur Synthese von aromatischen Olefinen im Labormaßstab ist die Heck-Reaktion, bei der lod- oder Bromaromaten und in Ausnahmefällen Chloraromaten mit Olefinen in Gegenwart von Palladiumkatalysatoren umgesetzt werden. Übersichten, die diese Methode beschreiben, findet man in R.F. Heck, Acc. Chem. Res. 1979, 12, 146; R.F. Heck, Org. React. 1982, 27, 345; R.F. Heck, Palladium Reagents in Synthesis, Academic Press, London 1985.

Die Katalysatoren, die für die Heck-Reaktion verwendet werden, sind Palladiumverbindungen. Obwohl sowohl Palladium(II)- als auch Palladium(O)-Komplexe bei Heck-Reaktionen eingesetzt werden, ist es doch allgemein akzeptiert, daß lediglich Palladium(O)-Verbindungen die eigentlichen Katalysatoren der Reaktion sind. Insbesondere formuliert man in der Literatur koordinativ ungesättigte 14-Elektronen Palladium(O)-Spezies, welche in der Regel mit schwachen Donorliganden wie Phosphanen stabilisiert werden.

Trotz der Vielzahl von Veröffentlichungen auf dem Gebiet der Heck-Reaktion sind bisher nur wenig Beispiele für eine technische Umsetzung dieser Methode bekannt. Dies ist darauf zurückzuführen, daß die beschriebenen Katalysatorsysteme häufig nur mit nicht ökonomischen Ausgangsmaterialien wie lodaromaten befriedigende katalytische Wechselzahlen ("turnover numbers") ergeben. Ansonsten müssen bei Bromaromaten und insbesondere bei Chloraromaten generell große Mengen an Katalysator - allgemein 1-5 Mol-% - zugesetzt werden, um technisch nutzbare Umsätze zu erzielen. Aufgrund der Komplexität der Reaktionsgemische ist zudem kein einfaches Katalysatorrecycling möglich, so daß auch die Katalysatorkosten in der Regel einer technischen Realisierung entgegenstehen.

DE-4421730 offenbart das bisher beste Verfahren unter Verwendung von Palladaphosphaindanen, sog. Palladacyclen, für die Heck-Reaktion. Es besteht in der Reaktion von Brom- und Chloraromaten mit Olefinen. Aktivierte Bromaromaten, wie z.B. 4-Bromacetophenon, 4-Brombenzaldehyd oder 4-lodbrombenzol, werden mit Katalysatormengen von 0,002 bis 0,01 mol% Palladium in Form von Palladaphosphaindan in Ausbeuten bis zu 100% umgesetzt. Weniger aktive Bromaromaten, wie z.B. Bromtoluol oder Brombenzol, werden mit Katalysatormengen von 2 mol% Palladium in Form von Palladaphosphaindan in Ausbeuten bis zu 96 % umgesetzt. Beim Umsatz von aktivierten Chloraromaten, wie z.B. Chloracteophenon, ist der Zusatz von Halogenidionen in Form der Salze, wie z.B. Lithiumbromid, notwendig um hohe Ausbeuten zu erreichen. So kann beim Umsatz 100 mmol 4-Chloracetophenon, 170 mmol 2-Ethylhexylacrylat, 110 mmol Natriumacetat, 10 mmol Lithiumbromid in 100 ml Dimethylacetamid mit 0,05 mmol Di-µ-acetato-bis-(o-(di-o-tolylphosphino)benzyl)dipalladium (II) (entspricht 0,1 mol% Palladium) als Katalysator nach 18 Stunden bei 130°C eine Ausbeute von 82 % E-4-Acetylzimtsäure-2-ethylhexylester erhalten werden. Da aufgrund der Komplexität der Reaktionsgemische auch beim Einsatz von Palladaphosphaindanen kein einfaches Katalysatorrecycling möglich ist, stehen im Fall der weniger aktiven Bromund Chloraromaten die Katalysatorkosten in der Regel einer technischen Realisierung entgegen. Im Fall der Chloraromaten ist zudem der Zusatz von Halogeniden bzw. Pseudohalogeniden ökologisch nachteilig, zumal diese keinen Beitrag zur Reaktion haben, sondern nur zur Stabilisierung der Palladaphosphaindane dienen soll.

Daher bestand ein Bedarf nach einem Verfahren, das die genannten Nachteile nicht aufweist, für die technische Durchführung geeignet ist und aromatische Olefine in hoher Ausbeute und Reinheit liefert.

Gegenstand der Erfindung ist ein Verfahren zur Herstellung von aromatischen Olefinen der Formel (I) 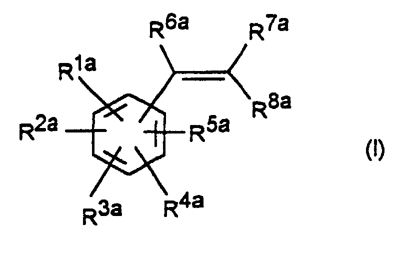 worin
- R^{1a} bis R^{5a}: unabhängig voneinander Wasserstoff, C₁-C₈-Alkyl, (C₁-C₈)-Alkoxy, (C₁-C₈)-Acyloxy, O-Phenyl, Phenyl, Fluor, Chlor, Brom, OH, NO₂, OSO₂CF₃, CN, COOH, CHO, SO₃H, SO₂R, SOR, NH₂, NH-(C₁-C₈)-Alkyl, N-(C₁-C₈)-Alkyl₂-, CHal₃, NHCO-(C₁-C₄)-Alkyl, N-(C₁-C₄)-Alkyl-CO-(C₁-C₄)-Alkyl, COO-(C₁-C₈)-Alkyl, CONH₂, CO-(C₁-C₈)-Alkyl, NHCOH, NCOO-(C₁-C₄)-Alkyl, CO-Phenyl, COO-Phenyl, CHCH-CO₂-(C₁-C₈)-Alkyl, CHCHCO₂H, PO-Phenylz, PO-(C₁-C₄)-Alkyl₂, OSO₂-Phenyl, OSO₂CH₃, wobei einer der Reste R^{1a} bis R^{5a} auch 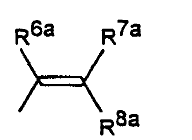 darstellen kann;
- R^{6a}: Wasserstoff, (C₁-C₈)-Alkyl, Phenyl, O-(C₁-C₈)-Alkyl, Fluor;
- R^{7a} und R^{8a}: unabhängig voneinander Wasserstoff, CN, CO₂H, CO₂-(C₁-C₈)-Alkyl, CONH₂, CONH-(C₁-C₄)-Alkyl, CON(C₁-C₄)-Alkyl₂, Fluor, CO₂-Phenyl, (C₁-C₈)-Phenyl, PO(Phenyl), PO-(C₁-C₄)-Alkyl₂, CO-Phenyl, CO-(C₁-C₄)-Alkyl, O-(C₁-C₄)-Alkyl, NH-(C₁-C₄)-Alkyl, PO₃H, SO₃H, SO₃-(C₁-C₄)-Alkyl, SO₂-(C₁-C₄)- Alkyl, O-Phenyl, (C₁-C₈)-Alkyl bedeuten, durch Umsetzung von Halogenaromaten der Formel (II) 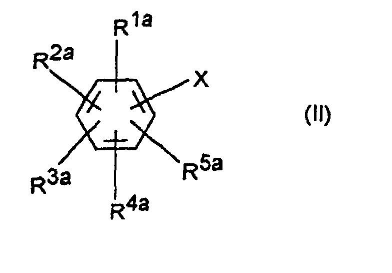 mit Olefinen der Formel (III) 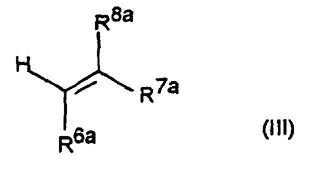 worin R^{1a} bis R^{8a} die oben angegebenen Bedeutungen besitzen, und worin X die für die Reste R^{1a} bis R^{5a} angegebene Bedeutung hat, dadurch gekennzeichnet, daß man als Katalysator eine Palladiumverbindung der Formel (IV) verwendet, 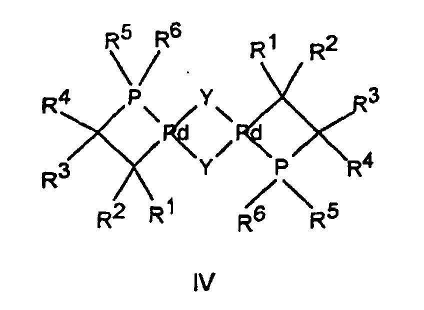 worin
- R¹, R²: unabhängig voneinander Wasserstoff, (C₁-C₄)-Alkyl, (C₃-C₁₂)-Cycloalkyl, (C₁-C₄)-Alkoxy, Fluor, N-(C₁-C₄)-(Alkyl)₂, CO₂-(C₁-C₄)-Alkyl, OCO-(C₁-C₄)-Alkyl oder Aryl;
- R³, R⁴, R⁵, R⁶: unabhängig voneinander (C₁-C₈)-Alkyl, (C₃-C₁₂)-Cycloalkyl, Aryl;
oder worin R¹ und R², R¹ oder R² und R³ oder R⁴, R³ und R⁴, R³ oder R⁴ und R⁵ oder R⁶, R⁵ und R⁶ zusammen einen aliphatischen Ring mit 4 bis 10 Kohlenstoffatomen bilden,
oder worin R⁵ und R⁶, R³ oder R⁴ und R⁵ oder R⁶ zusammen einen aromatischen Ring mit 5 bis 9 Kohlenstoffatomen bilden,
und
Y ein Anion einer anorganischen oder organischen Säure bedeutet.

Bevorzugt wird das Verfahren mit Verbindungen der Formel (IV) ausgeführt, worin
- R¹, R²: unabhängig voneinander Phenyl,
- R⁵, R⁶: unabhängig voneinander Phenyl, Naphthyl, Anthracenyl, die mit 1 bis 3 (C₁-C₄)-Alkyl- oder 1 bis 3 (C₁-C₄)-Alkoxy-Gruppen substituiert sein können,
und
- Y: Acetat, Propionat, Benzoat, Chlorid, Bromid, lodid, Fluorid, Sulfat, Hydrogensulfat, Nitrat, Phosphat,Tosylat, Mesylat, Trifluoromethansuifonat, Tetrafluoroborat, Acetylacetonat, Hexafluoracetylacetonat oder Pyrazolyl bedeuten.

Besonders bevorzugt wird das Verfahren mit Verbindungen der Formel (IV) ausgeführt, worin
- R⁵, R⁶: unabhängig voneinander o-Trifluoromethylphenyl, o-Trifluoromethyl-p-tolyl, o-Trifluoromethyl-p-methoxy-phenyl, o-Methoxy-phenyl, o,p-Dimethoxy-phenyl, o,o,p-Trimethoxy-phenyl, Anthracenyl, tert.-Butyl, n-Butyl, Isopropyl, Isobutyl, Cyclohexyl, 1-Methylcyclohexyl
bedeuten.

Insbesondere werden die Verbindungen:
Di-µ-acetato-bis[2-[bis(1,1-dimethylethyl)phosphino]-2-methylpropyl-C,P]dipalladium (II)
Di-µ-acetato-bis[2-[(1,1-dimethylethyl)-phenylphosphino]-2-methylpropyl-C,P]dipalladium (II)
Di-µ-chloro-bis[2-[bis(1,1-dimethylethyl)phosphino]-2-methylpropyl-C,P]dipalladium (II)
Di-µ-chloro-bis[2-[(1,1-dimethylethyl)-phenylphosphino]-2-methylpropyl-C,P]dipalladium (II)
Di-µ-bromo-bis[2-[bis(1,1-dimethylethyl)phosphino]-2-methylpropyl-C,P]dipalladium (II)
Di-µ-bromo-bis[2-[(1,1-dimethylethyl)-phenylphosphino]-2-methylpropyl-C,P]dipalladium (II)
für das Verfahren verwendet.

Als Lösungsmittel finden im allgemeinen inerte organische Lösungsmittel Verwendung. Gut geeignet sind dipolar aprotische Lösungsmittel wie Dialkylsulfoxide, N,N-Dialkylamide von aliphatischen Carbonsäuren oder alkylierte Lactame. Hierbei sind Dimethylsulfoxid, Dimethylacetamid, Dimethylformamid und N-Methylpyrrolidon bevorzugt.

Die Reaktion läuft bei Temperaturen von 20 bis 200°C ab, in vielen Fällen hat es sich bewährt, bei Temperaturen von 60 bis 180°C, bevorzugt 80 bis 150°C zu arbeiten.

Da bei der Reaktion HX abgespalten wird, ist es vorteilhaft, diese Säure durch Zusatz einer Base abzufangen. Dazu geeignet sind primäre, sekundäre oder tertiäre Amine wie Alkylamine, Dialkylamine, Trialkylamine, die alicyclisch oder offenkettig sein können, Alkali- oder Erdalkalisalze von aliphatischen oder aromatischen Carbonsäuren oder der Kohlensäure, wie Lithium, Natrium-, Kalium-, Calcium-, Magnesiumacetat und entsprechende Carbonate oder Hydrogencarbonate und Oxide oder Hydroxide der Alkali- oder Erdalkalimetalle, wie z.B. Lithium-, Magnesium- oder Calciumhydroxid und Calcium- oder Magnesiumoxid. Alle Basen können ebenfalls in Form wasserhaltiger Verbindungen eingesetzt werden, was den Vorteil hat, daß schwer wasserfrei darstellbare Basen einsetzbar sind.

Die eingesetzten Palladiumkatalysatoren werden in der Regel vor der eigentlichen Reaktion isoliert synthetisiert, sie können jedoch auch in situ erzeugt werden, ohne daß dadurch die anfängliche katalytische Aktivität gemindert wird. Bei längerer Reaktionführung erweisen sich die in situ erzeugten Mischungen (molares Verhältnis Pd:P = 1:1) jedoch als weniger stabil und führen häufig zur Palladiumabscheidung. Es muß daher bei in situ Mischungen mit einem Phosphan-Überschuß gearbeitet werden, welcher beim Einsatz der Palladaphosphacyclobutane entfällt.

Die eingesetzten oder sich bildenden Palladaphosphacyclobutane haben in der Regel einen dimeren Aufbau. Bei bestimmten Verbindungen (z.B. Y = Acetylaceton, Hexafluoracetylaceton) können jedoch auch monomere, oligomere oder polymere Strukturen vorliegen.

Während des Katalysezyklus wird durch Brückenspaltungsreaktionen mit anorganischen und organischen Nucleophilen die dimere Struktur aufgebrochen, so daß als eigentlich katalytisch aktive Spezies die einkernigen Komplexe der Formel (V) bzw. (VI) 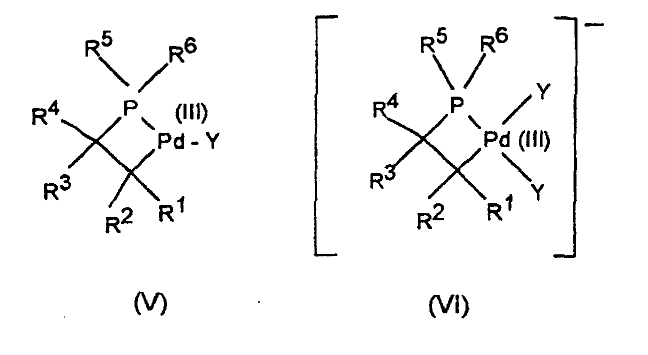 in Betracht zu ziehen sind. Die Komplexe der Formel (V) und (VI) stehen mit den tatsächlichen eingesetzten Dimeren im Austauschgleichgewicht und haben neutralen oder anionischen Charakter. Der einkernige Komplex der Formel (V) kann dabei gegebenenfalls weitere Donorliganden am Palladiumatom enthalten.

Der sehr vorteilhafte Verlauf der erfindungsgemäßen Reaktion war besonders überraschend, da nach dem Stand der Technik Palladiumkatalysatoren der Formel (IV) für die Durchführung der Heck-Reaktion nicht beschrieben sind.

Die als neue Katalysatorsysteme eingesetzten Palladphosphacyclobutane zeichnen sich durch sehr große Aktivität und unerwartet hohe Stabilität aus.

Die Stabilität der Palladaphosphacyclobutane in Lösung läßt sich durch Zusatz von Alkali-, Erdalkali- und Übergangsmetallsalzen der 6. bis 8. Nebengruppe erhöhen. Insbesondere der Zusatz von Halogeniden und Pseudohalogeniden (z.B. CN⁻) bewirken bei der Umsetzung von Chloraromaten signifikante Ausbeutesteigerungen (1 bis 100 %) und Standzeitverbesserungen des Katalysators. Geeignet sind auch Tri- und Tetraalkylammonium-Salze sowie entsprechende Phosphonium- und Arsonium-Salze.

Es können Turnover-Werte in der Größenordnung von 1.000.000 und mehr realisiert werden.

Aufgrund der Katalysatoraktivität und -stabilität ist es somit bei bestimmten Verfahren möglich, extrem kleine Mengen an Katalysator zu verwenden, so daß die Katalysatorkosten im Vergleich zum Stand der Technik sehr gering sind.

Außerdem bedingt der Einsatz kleinster Mengen an Katalysator ökologische Vorteile, da Abfallprodukte oder abfallerzeugende Aufarbeitungsverfahren vermieden werden.

Die nachstehenden Beispiele dienen zur näheren Erläuterung des erfindungsgemäßen Verfahrens

### Beispiel 1: Synthese des Katalysators

### Di-µ-acetato-bis[2-[bis(1,1-dimethylethyl)phosphino]-2-methylpropyl-C,P]dipalladiun (II)

5,10 g (22,7 mmol) Pd(OAc)₂ werden in 200 ml Toluol mit rotbrauner Farbe gelöst. Die Lösung wird mit 5,00 mg (24,7 mmol) Tri-(tert.-butyl)phosphan versetzt. Die sich rasch nach hellorange aufklarende Lösung wird 10 Minuten lang auf 70-80°C erhitzl und dann auf Raumtemperatur abgekühlt. Das Lösungsmittel wird im Vakuum entfernt. Nach Zugabe von 200 ml Hexan kristallisiert nach kurzer Zeit das Produkt, Di-µ-acetato-bis[2-[bis(1,1-dimethylethyl)phosphino]-2-methylpropyl-C,P]dipalladium, aus und wird abfiltriert. Man erhält 6,65 g (80 % d. Th.) bezogen auf Pd(OAc)₂) als weißgelben Feststoff (Schmp. > 200°C). Durch Umkristallisation aus Hexan und Filtration der Lösungen über Celite® kann das Produkt in Form weißgelber Kristallnadeln analysenrein gewonnen werden.

| Elementaranalyse: C₂₈H₅₈O₄P₂Pd₂ (733,51): | | |
|---|---|---|
| gef. | C, 45.6 %; | H, 7.7 %; |
| ber. | C, 45.85 %; | H, 7.97 %; |

¹H-NMR (300 MHz, CDCl₃): d =1.88 (3H, s, CH₃); 1.50 (d, 18H, CH₃, ⁴J(PH) = 14 Hz); 1.44 (d, 12H, CH₃, ⁴J(PH) = 15 Hz); 1.07 (2H, s_{breit}, 4H, CH₂);
¹³C{¹H}-NMR (75.4 MHz, -70 C, CD₂Cl₂): d = 181.5 (s, CH₃CO₂); 49.5 (s, PC, J(PC)= 20.1 Hz); 37.5 (s, PC, J(PC)= 10.6 Hz); 32.3 (s, CH₃, J(PC)= 2,9 Hz); 31.1 (s, CH₃); 24.7 (s, CH₃CO₂); 7.2 (s, CH₂, J(PC)= 33.6 Hz).
³¹P{¹H}-NMR (121.4 MHz, CDCl₃): = -8.5 (s).

### Vergleichsbeispiel 1

16 mmol 4-Bromanisol, 21 mmol n-Butylacrylat, 18 mmol Kaliumcarbonat werden in 10 ml Dimethylacetamid mit 0,08 µmol Di-µ-acetato-bis[-o-(di-otolylphosphino)benzyl]dipalladium (II) als Katalysator 16 Stunden bei 140°C gerührt.
Ausbeute: 17,7 % 4-Methoxyzimtsäure-n-butylester (GC-Analyse)

### Vergleichsbeispiel 2

16 mmol 4-Bromanisol, 21 mmol n-Butylacrylat, 18 mmol Kaliumcarbonat werden in 10 ml Dimethylacetamid mit 0,16 µmol Palladiumacetat und 0,16 µmol Tri-(tert.butyl)phosphan als Katalysator 17 Stunden bei 140°C gerührt.
Ausbeute: 13,8 % 4-Methoxyzimtsäure-n-butylester (GC-Analyse)

### Beispiel 2

16 mmol 4-Brornanisol, 21 mmol n-Butylacrylat, 18 mmol Kaliumcarbonat werden in 10 ml Dimethylacetamid mit 0,08 µmol Di-µ-acetato-bis[2-[bis(1,1-dimethylethyl)phosphino]-2-methylpropyl-C,P]dipalladium (II) als Katalysator 16 Stunden bei 140°C gerührt.
Ausbeute: 89,9 % 4-Methoxyzimtsäure-n-butylester (GC-Analyse)

### Vergleichsbeispiel 3

16 mmol 4-Bromanisol, 21 mmol n-Butylacrylat, 18 mmol Kaliumcarbonat werden in 10 ml Dimethylacetamid mit 1,6 µmol Palladiumacetat und 1,6 µmol Tri-(tert.butyl)phosphan als Katalysator 17 Stunden bei 140°C gerührt.
Ausbeute: 45,2 % 4-Methoxyzimtsäure-n-butylester (GC-Analyse)

### Beispiel 3

16 mmol 4-Bromanisol, 21 mmol n-Butylacrylat, 18 mmol Kaliumcarbonat werden in 10 ml Dimethylacetamid mit 0,8 µmol Di-µ-acetato-bis[2-[bis(1,1-dimethylethyl)phosphino]-2-methylpropyl-C,P]dipalladium (II) als Katalysator 24 Stunden bei 140°C gerührt.
Ausbeute: 99,2 % 4-Methoxyzimtsäure-n-butylester (GC-Analyse)

### Vergleichsbeispiel 4

10 mmol 4-Chlorbenzaldehyd, 15 mmol n-Butylacrylat, 15 mmol Natriumacetat, 2 mmol Tetrabutylammoniumbromid werden in 10 ml Dimethylacetamid mit 0,01 mmol Di-µ-acetato-bis[-o-(di-o-tolylphosphino)benzyl]dipalladium (II) als Katalysator 4 Stunden bei 140°C gerührt.
Ausbeute: 35,2% 4-Formylzimtsäurebutylester (GC-Analyse)

### Beispiel 4

10 mmol 4-Chlorbenzaldehyd, 15 mmol n-Butylacrylat, 15 mmol Natriumacetat, 2 mmol Tetrabutylammoniumbromid werden in 10 ml Dimethylacetamid mit 0,01 mmol Di-µ-chloro-bis[2-[bis(1,1-dimethylethyl)phosphino]-2-methylpropyl-C, P]dipalladium (II) als Katalysator 4 Stunden bei 140°C gerührt.
Ausbeute: 71,7 % 4-Formylzimtsäurebutylester (GC-Analyse)

### Vergleichsbeispiel 5

20 mmol Brombenzol, 25 mmol n-Butylacrylat und 25 mmol Kaliumcarbonat werden in 20 ml Dimethylacetamid mit 1,0 µmol Di-µ-acetato-bis[-o-(di-otolylphosphino)benzyl]dipalladium (II) als Katalysator 2 Stunden bei 140°C gerührt.
Ausbeute: 88,9% Zimtsäurebutylester (GC-Analyse)

### Vergleichsbeispiel 6

20 mmol Brombenzol, 25 mmol n-Butylacrylat und 25 mmol Kaliumcarbonat werden in 20 ml Dimethylacetamid mit 2,0 µmol Palladiumacetat als Katalysator 2 Stunden bei 140°C gerührt.
Ausbeute: 70,8% Zimtsäurebutylester (GC-Analyse)

### Beispiel 5

20 mmol Brombenzol, 25 mmol n-Butylacrylat und 25 mmol Kaliumcarbonat werden in 20 ml Dimethylacetamid mit 1,0 µmol Di-µ-chloro-bis[2-[bis(1,1-dimethylethyl)phosphino]-2-methylpropyl-C,P]dipalladium (II) als Katalysator 2 Stunden bei 140°C gerührt.
Ausbeute: 97,2% Zimtsäurebutylester (GC-Analyse)

### Beispiel 6

20 mmol Brombenzol, 25 mmol n-Butylacrylat und 25 mmol Kaliumcarbonat werden in 20 ml Dimethylacetamid mit 1,0 µmol Di-µ-acetato-bis[2-[bis(1,1-dimethylethyl)phosphino]-2-methylpropyl-C, P]dipalladium (II) als Katalysator 2 Stunden bei 140°C gerührt.
Ausbeute: 95,0% Zimtsäurebutylester (GC-Analyse)

### Vergleichsbeispiel 7

32 mmol 4-Bromacetophenon, 35 mmol n-Butylacrylat, 35 mmol Natriumacetat werden in 25 ml Dimethylacetamid 48 Stunden bei 140°C gerührt.
Ausbeute: 0% 4-Acetylzimtsäure-n-butylester (GC-Analyse)

### Beispiel 7

32 mmol 4-Bromacetophenon, 35 mmol n-Butylacrylat, 35 mmol Natriumacetat werden in 25 ml Dimethylacetamid mit 0,0016 µmol Di-µ-acetato-bis[2-[bis(1,1-dimethylethyl)phosphino]-2-methylpropyl-C,P]dipalladium (II) als Katalysator 48 Stunden bei 140°C gerührt.
Ausbeute: 98,8% 4-Acetylzimtsäure-n-butylester (GC-Analyse)

### Beispiel 8

20 mmol Chlorbenzol, 25 mmol n-Butylacrylat, 2 mmol Lithiumbromid und 25 mmol Kaliumcarbonat werden in 20 ml Dimethylacetamid mit 0,1 mmol Di-µ-acetato-bis[2-[bis(1,1-dimethylethyl]phosphino]-2-methylpropyl-C,P]dipalladium (II) als Katalysator 16 Stunden bei 140°C gerührt.
Ausbeute: 88,5% Zimtsäurebutylester (GC-Analyse)

Es wurden folgende Handelsprodukte verwendet:
Celite®/Aldrich Filterhilfsmittel auf SiO₂-Basis

## Patentansprüche

1. Verfahren zur Herstellung von aromatischen Olefinen der Formel (I) 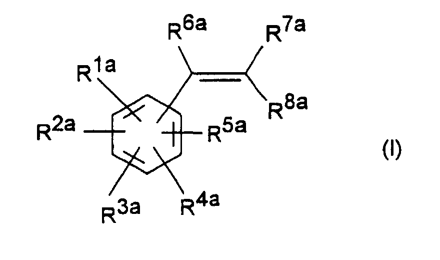 worin
R^{1a} bis R^{5a} unabhängig voneinander Wasserstoff, C₁-C₈-Alkyl, (C₁-C₈)-Alkoxy, (C₁-C₈)-Acyloxy, O-Phenyl, Phenyl, Fluor, Chlor, Brom, OH, NO₂, OSO₂CF₃, CN, COOH, CHO, SO₃H, SO₂R, SOR, NH₂, NH-(C₁-C₈)-Alkyl, N-(C₁-C₈)-Alkyl₂-, CHal₃, NHCO-(C₁-C₄)-Alkyl, N-(C₁-C₄)-Alkyl-CO-(C₁-C₄)-Alkyl, COO-(C₁-C₈)-Alkyl, CONH₂, CO-(C₁-C₈)-Alkyl, NHCOH, NCOO-(C₁-C₄)-Alkyl, CO-Phenyl, COO-Phenyl, CHCH-CO₂-(C₁-C₈)-Alkyl, CHCHCO₂H, PO-Phenyl₂, PO-(C₁-C₄)-Alkyl₂, OSO₂-Phenyl, OSO₂CH₃, wobei einer der Reste R^{1a} bis R^{5a} auch 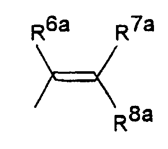 darstellen kann;
R^{6a} Wasserstoff, (C₁-C₈)-Alkyl, Phenyl, O-(C₁-C₈)-Alkyl, Fluor;
R^{7a} und R^{8a} unabhängig voneinander Wasserstoff, CN, CO₂H, CO₂-(C₁-C₈)-Alkyl, CONH₂, CONH-(C₁-C₄)-Alkyl, CON(C₁-C₄)-Alkyl₂, Fluor, CO₂-Phenyl, (C₁-C₈)-Phenyl, PO(Phenyl), PO-(C₁-C₄)-Alkyl₂, CO-Phenyl, CO-(C₁-C₄)-Alkyl, O-(C₁-C₄)-Alkyl, NH-(C₁-C₄)-Alkyl, PO₃H, SO₃H, SO₃-(C₁-C₄)-Alkyl, SO₂-(C₁-C₄)- Alkyl, O-Phenyl, (C₁-C₈)-Alkyl
bedeuten, durch Umsetzung von Halogenaromaten der Formel (II) 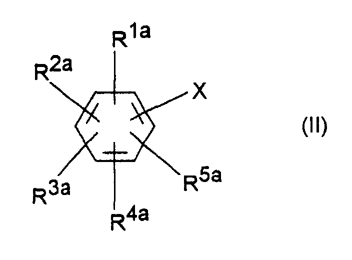 mit Olefinen der Formel (III) 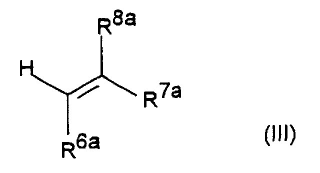 worin R^{1a} bis R^{8a} die oben angegebenen Bedeutungen besitzen, und worin X die für die Reste R^{1a} bis R^{5a} angegebene Bedeutung hat, **dadurch gekennzeichnet, daß** man als Katalysator eine Palladiumverbindung der Formel (IV) verwendet, 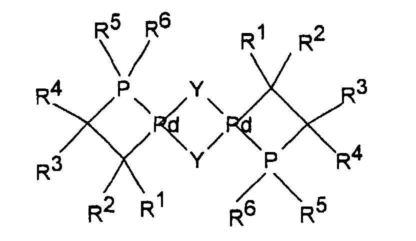 worin
R¹, R² unabhängig voneinander Wasserstoff, (C₁-C₄)-Alkyl, (C₃-C₁₂)-Cycloalkyl, (C₁-C₄)-Alkoxy, Fluor, N-(C₁-C₄)-(Alkyl)₂, CO₂-(C₁-C₄)-Alkyl, OCO-(C₁-C₄)-Alkyl oder Aryl;
R³, R⁴, R⁵, R⁶ unabhängig voneinander (C₁-C₈)-Alkyl, (C₃-C₁₂)-Cycloalkyl, Aryl;
oder worin R¹ und R², R¹ oder R² und R³ oder R⁴, R³ und R⁴, R³ oder R⁴ und R⁵ oder R⁶, R⁵ und R⁶ zusammen einen aliphatischen Ring mit 4 bis 10 Kohlenstoffatomen bilden,
oder worin R⁵ und R⁶, R³ oder R⁴ und R⁵ oder R⁶ zusammen einen aromatischen Ring mit 5 bis 9 Kohlenstoffatomen bilden,
und
Y ein Anion einer anorganischen oder organischen Säure bedeutet.

2. Verfahren nach Anspruch 1, **dadurch gekennzeichnet, daß** in Formel (IV)
R¹, R² unabhängig voneinander Phenyl,
R⁵, R⁶ unabhängig voneinander Phenyl, Naphthyl, Anthracenyl, die mit 1 bis 3 (C₁-C₄)-Alkyl- oder 1 bis 3 (C₁-C₄)-Alkoxy-Gruppen substituiert sein können,
und
Y Acetat, Propionat, Benzoat, Chlorid, Bromid, lodid, Fluorid, Sulfat, Hydrogensulfat, Nitrat, Phosphat,Tosylat, Mesylat, Trifluoromethansulfonat, Tetrafluoroborat, Acetylacetonat, Hexafluoracetylacetonat oder Pyrazolyl bedeuten.

3. Verfahren nach Anspruch 1 und/oder 2, **dadurch gekennzeichnet, daß** in Formel (IV)
R⁵, R⁶ unabhängig voneinander o-Trifluoromethylphenyl, o-Trifluoromethyl-p-tolyl, o-Trifluoromethyl-p-methoxy-phenyl, o-Methoxy-phenyl, o,p-Dimethoxyphenyl, o,o,p-Trimethoxy-phenyl, Anthracenyl, tert.-Butyl, n-Butyl, Isopropyl, Isobutyl, Cyclohexyl, 1-Methylcyclohexyl
bedeuten.

4. Verfahren nach einem oder mehreren der Ansprüche 1 bis 3, **dadurch gekennzeichnet, daß** als Katalysator die Verbindungen:
Di-µ-acetato-bis[2-[bis(1,1-dimethylethyl]phosphino]-2-methylpropyl-C,P]dipalladium (II),
Di-µ-acetato-bis[2-[(1,1-dimethylethyl)-phenylphosphino]-2-methylpropyl-C,P]dipalladium (II),
Di-µ-chloro-bis[2-[bis(1,1-dimethylethyl)phosphino]-2-methylpropyl-C,P]dipalladium (II),
Di-µ-chloro-bis[2-[(1,1-dimethylethyl)-phenylphosphino]-2-methylpropyl-C,P]dipalladium (II),
Di-µ-bromo-bis[2-[bis(1,1-dimethylethyl)phosphino]-2-methylpropyl-C,P]dipalladium (II) und
Di-µ-bromo-bis[2-[(1,1-dimethylethyl)-phenylphosphino]-2-methylpropyl-C,P]dipalladium (II)
eingesetzt werden.

5. Verfahren nach einem oder mehreren der Ansprüche 1 bis 4, **dadurch gekennzeichnet, daß** der Katalysator in situ hergestellt wird.

6. Verfahren nach einem oder mehreren der Ansprüche 1 bis 5, **dadurch gekennzeichnet, daß** als Lösungsmittel dipolar aprotische Lösungsmittel verwendet werden.

7. Verfahren nach Anspruch 6, **dadurch gekennzeichnet, daß** als Lösungsmittel Dialkylsulfoxide, N,N-Dialkylamide und/oder alkylierte Lactame verwendet werden.

8. Verfahren nach Anspruch 6, **dadurch gekennzeichnet, daß** als Lösungsmittel Dimethylsulfoxid, Dimethylacetamid, Dimethylformamid und/oder N-Methylpyrrolidon verwendet werden.

9. Verfahren nach einem oder mehreren der Ansprüche 1 bis 8, **dadurch gekennzeichnet, daß** die Reaktion bei Temperaturen von 20 bis 200°C durchgeführt wird.

10. Verfahren nach Anspruch 9, **dadurch gekennzeichnet, daß** die Reaktion bei Temperaturen von 60 bis 180°C durchgeführt wird.

11. Verfahren nach Anspruch 9, **dadurch gekennzeichnet, daß** die Reaktion bei Temperaturen von 80 bis 150°C durchgeführt wird.

12. Verfahren nach einem oder mehreren der Ansprüche 1 bis 11, **dadurch gekennzeichnet, daß** die bei der Reaktion entstehende Säure HX durch Zusatz einer Base abgefangen wird.

13. Verfahren nach Anspruch 12, **dadurch gekennzeichnet, daß** als Base Amine oder Alkali- oder Erdalkalimetallsalze von schwachen Säuren verwendet werden.

14. Verfahren nach Anspruch 12, **dadurch gekennzeichnet, daß** als Base Alkylamine oder die Carbonate, Hydrogencarbonate, Hydroxide, Oxide oder Acetate von Lithium, Natrium, Kalium, Calcium oder Magnesium eingesetzt werden.

15. Verfahren nach einem oder mehreren der Ansprüche 1 bis 14, **dadurch gekennzeichnet, daß** Salze von Halogeniden und Pseudohalogeniden der Alkali-, Erdalkalimetalle und Metalle der 6. bis 8. Nebengruppe zugesetzt werden.

16. Verfahren nach einem oder mehreren der Ansprüche 1 bis 15, **dadurch gekennzeichnet, daß** Tri- oder Tetraalkylammonium, -phosphonium oder - arsoniumsalze zugesetzt werden.

## Claims

1. A process for preparing aromatic olefins of the formula (I) 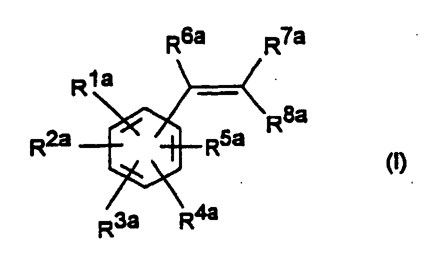 in which
R^{1a} to R^{5a} signify, independently of one another, hydrogen, C₁-C₈-alkyl, (C₁-C₈)-alkoxy, (C₁-C₈)-acyloxy, O-phenyl, phenyl, fluorine, chlorine, bromine, OH, NO₂, OSO₂CF₃, CN, COON, CHO, SO₃H, SO₂R, SOR, NH₂, NH-(C₁-C₈)-alkyl, N-(C₁-C₈)-alkyl₂-, CHal₃, NHCO-(C₁-C₄)-alkyl, N-(C₁-C₄)-alkyl-CO-(C₁-C₄) -alkyl, COO-(C₁-C₈)-alkyl, CONH₂, -CO-(C₁-C₈)-alkyl, -NHCOH, NCOO- (C₁-C₄) -alkyl, CO-phenyl, COO-phenyl, CHCH-CO₂-(C₁-C₈)-alkyl, CHCHCO₂H, PO-phenyl₂, PO-(C₁-C₄)-alkyl₂, OSO₂-phenyl, OSO₂CH₃, whereby one of the residues R^{1a} to R^{5a} may also represent 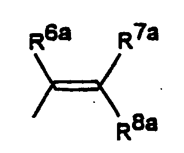
R^{6a} signifies hydrogen, C₁-C₈-alkyl, phenyl, O-(C₁-C₈)-alkyl, fluorine;
R^{7a} and R^{8a} signify, independently of one another, hydrogen, CN, CO₂H, CO₂-(C₁-C₉)-alkyl, CONH₂, CONH- (C₁-C₄) -alkyl, CON(C₁-C₄) -alkyl₂, fluorine, CO₂-phenyl, (C₁-C₈)-phenyl, PO(phenyl), PO-(C₁-C₄)-alkyl₂, CO-phenyl, CO-(C₁-C₄) -alkyl, O-(C₁-C₄)-alkyl, NH-(C₁-C₄)-alkyl, PO₃H, SO₃H, SO₃-(C₁-C₄)-alkyl, SO₂-(C₁-C₄)-alkyl, O-phenyl, (C₁-C₈)-alkyl,
by conversion of haloaromatics of the formula (II) 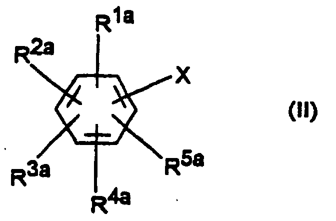 with olefins of the formula (III) 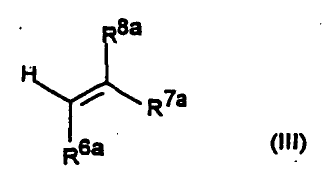 in which R^{1a} to R^{8a} have the meanings specified above, and in which X has the meaning specified for the residues R^{1a} to R^{5a}, **characterised in that** by way of catalyst use is made of a palladium compound of the formula (IV) 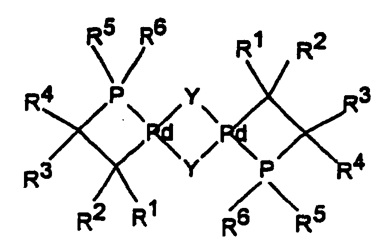 in which
R¹, R² signify, independently of one another, hydrogen, (C₁-C₄)-alkyl, (C₃-C₁₂)-cycloalkyl, (C₁-C₄)-alkoxy, fluorine, N-(C₁-C₄)-(alkyl)₂, CO₂-(C₁-C₄)-alkyl, OCO-(C₁-C₄)-alkyl or aryl;
R³, R⁴, R⁵, R⁶ signify, independently of one another, (C₁-C₈)-alkyl, (C₃-C₁₂)-cycloalkyl, aryl;
or in which R¹ and R², R¹ or R² and R³ or R⁴, R³ and R⁴, R³ or R⁴ and R⁵ or R⁶, R⁵ and R⁶ together form an aliphatic ring with 4 to 10 carbon atoms,
or in which R⁵ and R⁶, R³ or R⁴ and R⁵ or R⁶ together form an aromatic ring with 5 to 9 carbon atoms,
and
Y signifies an anion of an inorganic or organic acid.

2. Process according to claim 1, **characterised in that** in formula (IV)
R¹, R² signify, independently of one another, phenyl,
R⁵, R⁶ signify, independently of one another, phenyl, naphthyl, anthracenyl, which may be substituted by 1 to 3 (C₁-C₄)-alkyl groups or 1 to 3 (C₁-C₄)-alkoxy groups,
and
Y signifies acetate, propionate, benzoate, chloride, bromide, iodide, fluoride, sulfate, hydrogensulfate, nitrate, phosphate, tosylate, mesylate, trifluoromethanesulfonate, tetrafluoroborate, acetylacetonate, hexafluoroacetylacetonate or pyrazolyl.

3. Process according to claim 1 and/or 2, **characterised in that** in formula (IV)
R⁵, R⁶ signify, independently of one another, o-trifluoromethylphenyl, o-trifluoromethyl-p-tolyl, o-trifluoromethyl-p-methoxyphenyl, o-methoxyphenyl, o,p-dimethoxyphenyl, o,o,p-trimethoxyphenyl, anthracenyl, tert.-butyl, n-butyl, isopropyl, isobutyl, cyclohexyl, 1-methylcyclohexyl.

4. Process according to one or more of claims 1 to 3, **characterised in that** the compounds:
di-µ-acetato-bis[2-[bis(1,1-dimethylethyl)phosphino]-2-methylpropyl-C,P]dipalladium(II),
di-µ-acetato-bis[2-[(1,1-dimethylethyl)phenylphosphino]-2-methylpropyl-C,P]dipalladium(II),
di-µ-chloro-bis[2-[bis(1,1-dimethylethyl)phosphino]-2-methylpropyl-C,P]dipalladium(II),
di-µ-chloro-bis[2-[(1,1-dimethylethyl)phenylphosphino]-2-methylpropyl-C,P]dipalladium(II),
di-µ-bromo-bis[2-[bis(1,1-dimethylethyl)phosphino]-2-methylpropyl-C,P]dipalladium(II) and
di-µ-bromo-bis[2-[(1,1-dimethylethyl)phenylphosphino]-2-methylpropyl-C,P]dipalladium(II)
are employed as catalyst.

5. Process according to one or more of claims 1 to 4, **characterised in that** the catalyst is produced in situ.

6. Process according to one or more of claims 1 to 5, **characterised in that** dipolar aprotic solvents are used as solvent.

7. Process according to claim 6, **characterised in that** dialkyl sulfoxides, N,N-dialkyl amides, and/or alkylated lactams are used as solvent.

8. Process according to claim 6, **characterised in that** dimethyl sulfoxide, dimethyl acetamide, dimethyl formamide and/or N-methyl pyrrolidone are used as solvent.

9. Process according to one or more of claims 1 to 8, **characterised in that** the reaction is carried out at temperatures from 20 °C to 200 °C.

10. Process according to claim 9, **characterised in that** the reaction is carried out at temperatures from 60 °C to 180 °C.

11. Process according to claim 9, **characterised in that** the reaction is carried out at temperatures from 80 °C to 150 °C.

12. Process according to one or more of claims 1 to 11, **characterised in that** the acid HX arising in the course of the reaction is intercepted by addition of a base.

13. Process according to claim 12, **characterised in that** amines or alkali-metal salts or alkaline-earth-metal salts of weak acids are used as base.

14. Process according to claim 12, **characterised in that** alkylamines or the carbonates, hydrogencarbonates, hydroxides, oxides or acetates of lithium, sodium, potassium, calcium or magnesium are employed as base.

15. Process according to one or more of claims 1 to 14, **characterised in that** salts of halides and pseudohalides of the alkali metals, alkaline-earth metals and metals pertaining to the 6th to 8th Subgroup are added.

16. Process according to one or more of claims 1 to 15, **characterised in that** tri- or tetraalkylammonium, tri- or tetraalkylphosphonium or tri- or tetraalkylarsonium salts are added.

## Revendications

1. Procédé de préparation d'oléfines aromatiques de formule (I) : 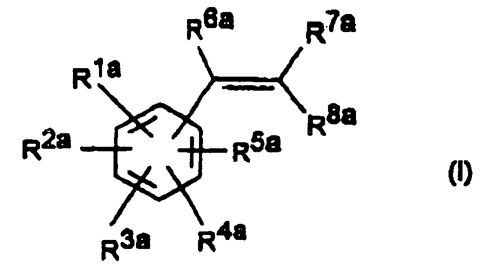 dans laquelle
R^{1a} à R^{5a} indépendamment les uns des autres signifient un alkyle en C₁-C₈, un alkoxy en C₁-C₈, un acyloxy en C₁-C₈, un O-phényle, un phényle, un fluor, un chlore, un brome, OH, NO₂, OSO₂CF₃, CN, COOH, CHO, SO₃H, SO₂R, NH₂, NH (alkyle en C₁-C₈), un N (alkyle en C₁-C₈)₂, C(Hal)₃, NHCO (alkyle en C₁-C₄), un N(alkyle en C₁-C₄)-CO-(alkyle en C₁-C₄), un COO(-alkyle en C₁-C₈), CONH₂, CO-(alkyle en C₁-C₈), NHCOH, NCOO-(alkyle en C₁-C₄), un CO-phényl, COOphényl, CHCH - CO₂ (alkyl en C1-C₈), CHCH CO₂H, PO-(phényle)₂, PO(alkyle en C₁-C₄)₂, OSO₂-phényle, OSO₂CH₃, dans laquelle un des restes R^{1a} à R^{5a} peut aussi représenter : 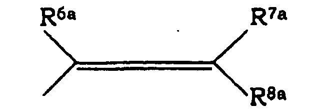 R^{6a} signifie de l'hydrogène, un (alkyle en C₁-C₈), un phényle, un O-(alkyle en C₁-C₈), un fluor ; R^{7a} et R^{8a} indépendamment l'un de l'autre signifient de l'hydrogène, CN, CO₂H, CO₂-(alkyle en C₁-C₈), CONH₂, CONH-(alkyle en C₁-C₄), CON-(alkyle en C₁-C₄)₂, un fluor, un CO₂-phényle, un phényle en C₁-C₈), un PO-(phényle), un PO (alkyle en C₁-C₄)₂, un CO-phényle, un CO-(alkyl en C₁-C₄), un O-(alkyle en C₁-C₄), un NH-(alkyle en C₁-C₄), un PO₃H, un SO₃H, un SO₃-(alkyle en C₁-C₄), un SO₂-(alkyle en C₁-C₄), un O-phényle, un (alkyle en C₁-C₈), par réaction de composés aromatiques halogénés de formule (II) : 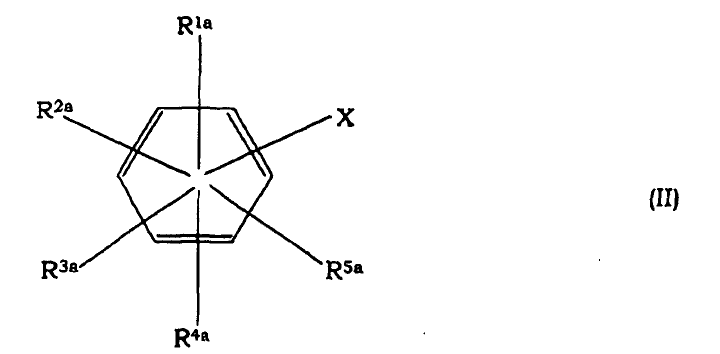 avec des oléfines de formule (III) : 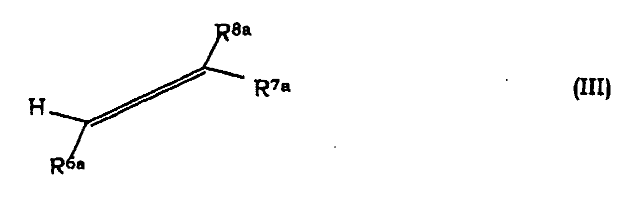 dans lesquelles R^{1a} à R^{8a} possèdent les significations indiquées ci-dessus
et dans lesquelles X a la signification indiquée pour les restes R^{1a} à R^{5a},
**caractérisé en ce qu'**
on utilise comme catalyseur un composé du palladium de formule IV : 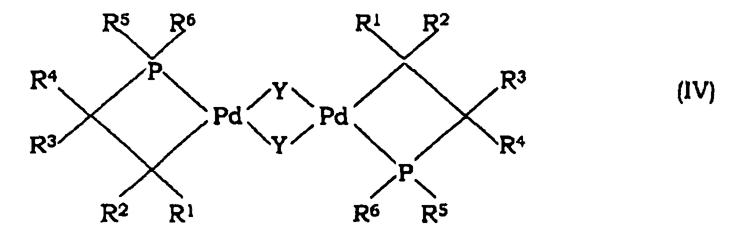 dans laquelle R¹ et R² indépendamment l'un de l'autre signifient de l'hydrogène, un (alkyle en C₁-C₄), un cyclo-(alkyle en C₃-C₁₂), un alkoxy en C₁-C₄, un fluor, N-(alkyle en C₁-C₄)₂, un CO₂-(alkyle en C₁-C₄), un OCO-(alkyl en C₁-C₄), ou un aryle,
R³ et R⁴, R⁵ et R⁶ indépendamment les uns des autres représentant un (alkyle en C₁-C₈), un cyclo-(alkyle en C₃-C₁₂), un aryle ;
ou dans laquelle R¹ et R², R¹ ou R² et R³ ou R⁴, R³ et R⁴, R³ ou R⁴ et R⁵
ou R⁶, R⁵ et R⁶ forment ensemble un cycle aliphatique ayant de 4 à 10 atomes de carbone,
ou dans laquelle R⁵ et R⁶, R³ ou R⁴ et R⁵ ou R⁶, ensemble forment un cycle aromatique ayant de 5 à 9 atomes de carbone,
et Y signifie un anion d'un acide minéral ou organique.

2. Procédé selon la revendication 1,
**caractérisé en ce que**
dans la formule (IV),
R¹ et R² indépendamment l'un de l'autre signifient un phényle,
R⁵ et R⁶ indépendamment l'un de l'autre signifient un phényle, un naphtyle, un anthracényle, qui peuvent être substitués par de 1 à 3 groupes (alkyle en C₁ à C₄) ou par de1 à 3 groupes (alkoxy en C₁-C₄),
et,
Y signifie un acétate, un propionate, un chlorure, un bromure, un iodure, un fluorure, un sulfate, un hydrogéno-sulfate, un nitrate, un phosphate, un tosylate, un mesylate, un tri-fluoro-méthane sulfonate, un tétra-fluoro-borate, un acétyl-acétonate, un hexa-fluoro-acétyl-acétonate, ou un pyrazolyle.

3. Procédé selon la revendication 1 et/ou 2,
**caractérisé en ce que**
dans la formule (IV) R⁵ et R⁶ indépendamment l'un de l'autre représentent un o-tri-fluoro-méthyl-phényle, un o-tri-fluoro-méthyle-p-tolyle, un o-tri-fluoro-méthyle-p-méthoxy-phényle, un o-méthoxy-phényle, un o,p-diméthoxy-phényle, un o,o,p-triméthoxy-phényle, un anthracinyle, un terbutyl, un n-butyle, un isopropyle, un isobutyle, un cyclohexyle, un 1-méthyl-cyclohexyle.

4. Procédé selon l'une ou plusieurs des revendications 1 à 3,
**caractérisé en ce que**
comme catalyseur on met en oeuvre les composés :
- d-µ-acétato-bis-[2-[bis(1,1-diméthyl)éthyl-phosphino]-2-méthyl-propyl-C,P] di-palladium (II),
- di-µ-acétato-bis-[2-[(1,1-diméthyl-éthyl)-phénylphosphino]-2-méthyl-propyl-C,P] di-palladium (II),
- di-µ-chloro-bis-[2-[bis-(1,1-diméthyl-éthyl)-phosphino]-2-méthyl-propyl-C,P] di-palladium (II),
- di-µ-chloro-bis-[2-[(1,1-diméthyl-éthyl)-phénylphosphino]-2-méthyl-propyl-C,P] di-palladium (II),
- di-µ-bromo-bis-[2-(bis-(1,1-diméthyl-éthyl)-phosphino]-2-méthyl-propyl-C,P] di-palladium (II), et,
- di-µ-bromo-bis-[2-[(1,1-diméthyl-éthyl)-phénylphosphino]-2-méthyl-propyl-C,P] di-palladium (II).

5. Procédé selon l'une ou plusieurs des revendications 1 à 4,
**caractérisé en ce que**
le catalyseur est produit "in situ".

6. Procédé selon l'une ou plusieurs des revendications 1 à 5,
**caractérisé en ce que**
comme solvants on utilise des solvants aprotiques dipolaires.

7. Procédé selon la revendication 6,
**caractérisé en ce que**
comme solvants on utilise des di-alkyl-sulfoxydes, des N,N-di-alkyl-amides et/ou des lactames alkylés.

8. Procédé selon la revendication 6,
**caractérisé en ce que**
comme solvants on utilise le di-méthyl-sulfoxyde, le di-méthyl-acétamide, le di-méthyl-formamide et/ou la N-méthyl-pyrrolidone.

9. Procédé selon l'une ou plusieurs des revendications 1 à 8,
**caractérisé en ce que**
la réaction est exécutée à des températures allant de 20 à 200 °C.

10. Procédé selon la revendication 9,
**caractérisé en ce que**
la réaction est exécutée à des températures allant de 60 à 180 °C.

11. Procédé selon la revendication 9,
**caractérisé en ce que**
la réaction est exécutée à des températures allant de 80 à 150 °C.

12. Procédé selon l'une ou plusieurs des revendications 1 à 11,
**caractérisé en ce que**
l'acide HX qui se forme au cours de la réaction est capté par addition d'une base.

13. Procédé selon la revendication 12,
**caractérisé en ce qu'**
on utilise comme base des aminés ou des sels de métal alcalins ou alcalino-terreux d'acide faible.

14. Procédé selon la revendication 12,
**caractérisé en ce qu'**
on met en oeuvre comme base des alkyl-amines ou les carbonates, les hydrogénocarbonates, les hydroxydes, les oxydes, ou les acétates de lithium, de sodium, de potassium, de calcium ou de magnésium.

15. Procédé selon l'une ou plusieurs des revendications 1 à 14,
**caractérisé en ce qu'**
on ajoute des sels d'halogénures ou de pseudo-halogénures de métal alcalino-terreux et de métaux des 6^{ème} et 8^{ème} sous-groupes.

16. Procédé selon l'une ou plusieurs des revendications 1 à 15,
**caractérisé en ce qu'**
on ajoute des sels de tri- ou de tétra-alkyl-ammonium, phosphonium, ou arsonium.
